# EUROPEAN PATENT SPECIFICATION

(11) **EP 3 243 520 B1**
(45) Date of publication and mention of the grant of the patent: **10.06.2020**
(21) Application number: 16203662.8
(22) Date of filing: 13.12.2016
(51) Int. Cl.: A23L 17/60, A61K 36/05, C12N 1/00, C12N 1/12, A23L 33/10, A21D 2/36, A23K 10/30, A23K 50/80, A23L 33/105, A23L 33/135, C12N 1/02, C12R 1/89

(54) **ALGAE-BASED FOOD FORMULATION, BREAD-MAKING, BAKERY AND CONFECTIONERY PRODUCTS CONTAINING IT, METHOD FOR OBTAINING THEREOF AND ITS USE**
ALGENBASIERTE LEBENSMITTELFORMULIERUNG, BROTHERSTELLUNG, BÄCKEREI- UND KONDITOREIPRODUKTE DAMIT, VERFAHREN ZUR HERSTELLUNG DAVON UND DEREN VERWENDUNG
FORMULATION ALIMENTAIRE À BASE D'ALGUE, FABRICATION DE PAIN, PRODUITS DE BOULANGERIE ET DE PÂTISSERIE LA CONTENANT, SON PROCÉDÉ D'OBTENTION ET SON UTILISATION

(30) Priority: 13.05.2016 ES 201630626
(43) Date of publication of application: 15.11.2017
(73) Proprietor: Juan y Juan Industrial, S.L. Unipersonal, 46720 Villalonga (Valencia) (ES)
(72) Inventor: JUAN FERNÁNDEZ, Rafael, 46720 VILLALONGA (Valencia) (ES)
(74) Representative: Ungria López, Javier

(56) References cited:
- WO-A1-2014/148943
- DE-A1- 10 029 743
- ES-A1- 2 378 102
- US-A1- 2010 297 296
- DATABASE WPI Week 200650 Thomson Scientific, London, GB; AN 2006-485078 XP002770613, & JP 2006 180868 A (KYOTO EIYO CO LTD) 13 July 2006 (2006-07-13)
- MÓNICA FRADIQUE ET AL: "Incorporation of Chlorella vulgaris and Spirulina maxima biomass in pasta products. Part 1: Preparation and evaluation", JOURNAL OF THE SCIENCE OF FOOD AND AGRICULTURE, vol. 90, no. 10, 15 August 2010 (2010-08-15), pages 1656-1664, XP055027243, ISSN: 0022-5142, DOI: 10.1002/jsfa.3999

## Description

### TECHNICAL FIELD OF THE INVENTION

This invention belongs to the field of the food industry, specifically applied to the manufacture of bakery and confectionery products.

### BACKGROUND OF THE INVENTION

In general terms, eating habits and the sufficient provision of all the nutritional requirements determine to a great degree the likelihood of suffering from certain disorders and/or recovering from them. From this perspective, the food industry has witnessed a revolution which has changed and will continue to change what we eat in the future because of the appearance of foods called functional or nutraceutical foods, defined as "any food or ingredient thereof which provides a proven benefit for human health".

The current trend in nutrition is to accentuate the importance of daily habits in which the rational choice of foods is based not only on the nutritional composition of those foods but also on their properties, some of them associated with the search for a healthy lifestyle. This means that the market is leaning ever more towards choosing products which help with health care, such as those which prevent illnesses, improve the body's functioning, prevent ageing and are more natural. There is also a preference for choosing products which do not require the investment of much available time, allowing other activities to be carried out.

The notion of a balanced diet is a fundamental concept, the result of a variety of research into nutrition relating nutrients to development, growth and maintenance of the organism and quality of life. In earlier times, it was considered that a balanced diet from the nutritional point of view was one which prevented deficiencies. Nowadays, this concept represents consuming an optimum diet based on foods which promote health and reduce the risk of chronic illnesses related to what we eat. For this reason, there are nutritional guidelines which advise the consumption of foods or food components of interest to public health, and also recommend avoiding excessive consumption of certain nutrients. At the beginning of the 21^{st} century the industrialised countries face new challenges: an enormous increase in the cost of healthcare, greater life expectancy, as we have said, increase in scientific knowledge, the appearance of new technologies and major changes in lifestyle.

In this sense, the concept of "functional food" expresses explicitly that foods and food components can have a beneficial influence on physiological functions by improving the state of wellbeing and health, and reducing the risk of illness. The notion of functional food is also designed to stimulate research into nutrition to support and validate the development of new foods and food components. Consequently, this new trend has seen the growth of so-called *Novel Foods* which are broadly defined as those foods which have not previously been eaten by humans and cannot be considered as obtained via a minor variation of the process used to obtain conventional foods.

In general, the difficulty in breaking down effectively the structures of the food also prevents the correct extraction of vitamins, minerals and essential fatty acids. It must be considered that the loss of function of the different muscle layers of the digestive tract affects the processes significantly and, consequently, work must be carried out on other points deriving from food. However, in addition to the adaptations in terms of composition and bioavailability of nutrients in certain formulas, also of fundamental interest is the consideration of the organoleptic characteristics of the product, which encourage chewing while at the same time improving the appetite with attractive aromas and flavours. Stimulation with regard to aromas, colours and flavours could make a great improvement to a correct diet, as the perception thresholds are usually increased and, with them, the lack of appetite for certain food groups.

The different aspects involved in the development of new products based on the different approaches referred to above include:
- Action on digestive difficulties (pre-hydrolysed foods, addition of enzymes, etc.)
- Improved absorption processes (channel activators, probiotics, prebiotics, etc.)
- Stimulants for the physical digestive process (peristalsis, etc.)
- Monitoring of the glycaemic index (modifying the speed of digestion/absorption of carbohydrates, etc.)
- Improved availability of vitamins and minerals.
- Organoleptic improvements in products (adapting aromas and flavours).
- Enrichment of the product according to the consumer's needs.
- Complete diets after the assessment of the requirements.
- Addition of functional compounds.

There is, thus, a need to find new sources of foods which can provide basic nutrients for human beings which are a market alternative to traditional raw materials, and which offer a reformulation of food products to achieve a substantial improvement in their nutritional value, without diminishing the organoleptic quality.

Amongst the possible forms of action, the algae market represents an area of expansion given their undeniable nutritional characteristics which, increasingly, are attributed functional properties to combat different pathologies derived from new lifestyles (antioxidants, anti-inflammatories, immunopotentiators, etc.) and used as a new source of highly functional ingredients, of nutrients and with components of technological interest. The recent increase in studies relating to the effects derived from the ingestion of, or exposure to, these microorganisms has had an influence on the increasing presence of enriched new products which can be seen on the current market. Said products are part of different ranges varying from food supplements or lotions to foodstuffs in the strict sense of the word (energy bars, biscuits, snacks, etc.).

The algae used include, notably, microalgae of the *Chlorella* genus, more specifically the species *Chlorella vulgaris,* which is a green, unicellular alga with a high chlorophyll content. A variety of studies have analysed its role as a functional product, given that it is a source of proteins, lipids, vitamins, minerals, carotenoids and other pigments (Ay, 1991), and contains high ratios of essential amino acids (Borowitzka, 1988; Schubert, 1988). In addition, it is an alga whose consumption has been deemed safe by the FDA (GRAS status). These organisms are currently used for a variety of purposes in the food market, particularly the Oriental market, including the manufacture of nutraceutical food supplements to supplement shortages or deficits of nutrients in the form of tablets, capsules or powders; their use as an ingredient in a variety of formulations to nutritionally enrich the end product, their adaptation for animal feeds, their use in aquaculture, and in other activities where the nutritional aspect is not the aim, such as the obtaining of energy. However, as is usually the case with algae-based food additives or ingredients, this type of functional food is used in a lyophilized or dry form (see JP 2006-180868 A), in powder or another form of presentation, which makes it easier to handle (and in many cases, its ingestion in the form of pills or similar). However, the preparation of lyophilized or dried algae involves an extra energy cost which does not always make the process economically advantageous, as the inventors have noted in the case of the species *Chlorella vulgaris* for industrial bread-making, bakery and confectionery products, and eliminates the liquid content of the algae which might have a potential value in the food product made from them.

In this regard, it should be emphasised that a major part of the difficulty in conceiving the preparation of this type of algae-based nutraceutical formulations, such as those of the species in question, lies in the fact that the final food products must not be deficient in organoleptic qualities compared to the conventional products on the market, to facilitate their acceptance by consumers, whereas the alga by nature has peculiar properties (smell, taste, etc.) which might condition completely those of the end product. For this reason, its incorporation as an ingredient may not be as obvious as it seems. In other words, given previous experience, consideration might be given to masking some properties of the alga to encourage its acceptance and facilitate its introduction into the market, particularly in a western society which is less accustomed to these products, and even less so when it comes to incorporating this ingredient in a conventional product such as industrial bakery; given that *Chlorella* has a peculiar smell (because it is an alga) compared to conventional food ingredients, there is a need to develop the food so that this aspect does not have a negative effect on consumption. At the same time, said microalga which is part of the formulation to be added must maintain as far as possible the characteristics of the food products to which it is added, such as, for example, the ability to ferment and the ability of the dough prepared in bakery to retain gas, while also maintaining the maximum number of bioavailable nutrients. Without diminishing the above, the microalgae must be able to provide the micronutrients necessary to meet the objectives sought by adding them, but without the ingredient being harmed by the treatments to which the product is subjected in its preparation and commercialisation, owing mainly to the cooking processes to which it is subjected. On this point, it is known that the wall of the microalgae is very resistant, which might affect the availability of the substances it contains to be assimilated by the organism and to provide the intended nutritional advantages.

To solve these problems simultaneously, there are no conventional methods developed in the market which favour the introduction of algae into the food products as a nutritional component, thereby reducing its current consumption to drugstore products. Nevertheless, some attempts to successfully use or include *Chlorella* in edible formulations are disclosed in the prior art. Document WO 2014/148943 A1 discloses the use of a *Chlorella* plankton strain as dietary biologically active supplement; whereas ES 2378102 A1 relates to a method for preparing spreadable products containing *Chlorella* (pyrenoidosa); and US 2010/297296 A discloses the preparation of baked goods with microalgae from *Chlorella.*

Following this line, and in light of the previous considerations on the prior art in the health food area, this invention proposes the development of food products, particularly in the area of bread-making, based on formulations of biomass of this alga but in a non-lyophilised state (fresh) which gives the food the healthy properties of the *Chlorella* without altering the fundamental properties of the product into which they are incorporated. Definitively, the purpose of the invention is to design and produce a range of bread-making products (industrial bakery and confectionery) prepared from microalgae with functional properties; it also attempts to introduce a raw material with functional properties into foods which, because of their formulation, cannot provide the consumer with said benefits.

### DESCRIPTION OF THE INVENTION

This invention refers to a food formulation based on microalgae of the *Chlorella* genus, particularly the species *Chlorella vulgaris,* specifically a food formulation comprising fresh microalgae in the form of paste of the *Chlorella vulgaris* species CCAP 211/11D in a proportion between 14% and 19% of the total weight of the formulation, with a degree of moisture measured at room temperature of between 81% and 86%, both inclusive. This formulation constitutes an ingredient or food additive based on fresh algae of the aforementioned species, which can be incorporated into the preparation of foods, particularly bread-making, industrial bakery and confectionery products.

"Fresh" microalgae are understood to be those cultivated in a liquid medium, harvested, subjected to a means of mechanical solid-liquid separation (for example, preferably by centrifuging) to give them the paste consistency, and incorporated into the formulation in their natural state, with no lyophilisation or drying treatment. It would be possible to lyophilise the microalgae to prepare the formulation after harvesting of the culture, although in principle this is of no interest, for two reasons. Firstly, to avoid having to carry out a lyophilisation process, which makes the manufacturing process more expensive, as it has been shown that the microalgae can be used in their natural, fresh state. Secondly, because the fact that the fresh algae are present in the formulation in the form of moist paste and not lyophilised is an advantage, since the formulation will facilitate the incorporation into the food product, because the water content favours the binding of the ingredients.

The formulation may contain microalgae of the species *Chlorella vulgaris* selected from one of the following varieties or strains:
- *Chlorella vulgaris Beijerinck (1980),* deposited in 1892 in the UK Culture Collection of Algae and Protozoa (CCAP), address SAMS Research Services Ltd., Scottish Marine Institute, OBAN, Argyll PA37 1QA Scotland, United Kingdom by (*Beijerinck*), from Freshwater (Delft, Holland, Netherlands), having received the CCAP deposit number 211/11B;
- *Chlorella vulgaris fo. viridis (Chodat) Fott* & *Novakova (1969)*, deposited in the UK Culture Collection of Algae and Protozoa (CCAP), address SAMS Research Services Ltd., Scottish Marine Institute, OBAN, Argyll PA37 1QA Scotland, United Kingdom by (Chodat), from Freshwater, having received the CCAP deposit number CCAP 211/12;
- *Chlorella vulgaris Beijerinck (1890),* deposited in 1946/7 in the UK Culture Collection of Algae and Protozoa (CCAP), address SAMS Research Services Ltd., Scottish Marine Institute, OBAN, Argyll PA37 1QA Scotland, United Kingdom by (von Witsch), from Freshwater; Gottingen, Germany, having received the CCAP deposit number 211/19;
- *Chlorella vulgaris Beijerinck (1890),* deposited in 1890 in the UK Culture Collection of Algae and Protozoa (CCAP), address SAMS Research Services Ltd., Scottish Marine Institute, OBAN, Argyll PA37 1QA Scotland, United Kingdom by (Cassie), from Freshwater; Lake Pupuke, Auckland, New Zealand, having received the CCAP deposit number 211/52;
- *Chlorella vulgaris fo. tertia Fott* & *Novakova (1969)*, deposited in 1938 in the UK Culture Collection of Algae and Protozoa (CCAP), address SAMS Research Services Ltd., Scottish Marine Institute, OBAN, Argyll PA37 1QA Scotland, United Kingdom by (Brannon 1938), from Freshwater; Madison, Wisconsin, USA, having received the CCAP deposit number CCAP 211/11D;
- *Chlorella vulgaris Beijerinck,* deposited on 26/05/2008 in the Spanish Algae Bank, address Muelle de Taliarte, s/n 35214- Telde, Gran Canaria (Spain), having received the deposit number BEA0753B. Collector Aitor Alonso Gonzalez (26/05/2008), Isolator V. Crux Alamo (11/05/2012), Depositor A. Laza-Martinez (10/11/2011).

The preferred selection of these strains was made on the basis of their excellent behaviour in terms of growth capacity (and consequently of formulation production after harvesting), and their better nutritional profile. In the invention, the food formulation contains the strain *Chlorella vulgaris fo. Tertia,* with deposit number CCAP 211/11D because of the improved culture yield, which provides a greater quantity of microalgae.

It has been shown that this formulation, understood as an additive or matrix which will be incorporated in the food composition, has properties which allow nutritionally complete, balanced foods to be obtained, which also have functional properties derived from the use of microalgae in their formulation; these will have a positive influence on the response of the consumer's immune system, and are also attractive for the public for whom they are intended. The formulation described is notable for the fact that it preserves the functional properties of the microalgae. Specifically, the food formulation contains minerals, vegetable proteins with high biological value, essential amino acids, polyunsaturated omega-3 fatty acids and vitamins A, B (B2, B3, B5, B6 and B12), C, E, iron and antioxidants which are found amongst the carotenoids and chlorophylls in the microalgae. This formulation or paste is thus an exceptional source of nutrients and micronutrients of high added value, to be used in the food industry. It was also shown, when analysing the functional and bioavailability effects exposed in Example 4 for young zebra fish that, despite the resistance of the alga cell wall, there was a clear absorption of its components in the organism, given that there were significant effects on the modifications of expression in metabolic routes of the individuals. Therefore, even though said wall is initially resistant, it seems that the digestion process makes it accessible, probably because of the action of different enzymes and juices which are able to break it down; consequently, it is not necessary, as believed, to carry out the lysis or breakdown of the algae cell wall, which might lead to a significant change in its properties.

As mentioned previously, the microalgae are in paste form because, after harvesting, the culture is subjected to a process of mechanical solid-liquid separation, which is particularly and preferably a process of centrifugation. In this way, 1000 litres of harvested aqueous culture of microalgae give between 7 and 10 kilograms of algae culture in the form of moist paste. The fact that the microalgae (and, by extension, the food formulation which contains them) are presented in moist paste form constitutes an important advantage because it considerably reduces its cost and greatly facilitates its manageability. It also makes it easier to incorporate it in the food product, thus encouraging the binding of the ingredients.

"Moist/Moisture" should be understood to mean the water content of the microalgae, measured at room temperature, where "room temperature" should be understood, in turn, to be a temperature of between 25°C-30°C. Preferably, the degree of moisture is around 83%. "Around 83%" should be understood to mean that the degree of moisture is preferably between 81% and 86%, and in the most preferred case, between 82% and 84%, both inclusive.

In the most preferred particular case, the food formulation consists exclusively of fresh microalgae of the species *Chlorella vulgaris,* in other words, it is its sole component in addition to the moisture content: a paste consisting exclusively of fresh microalgae with a high degree of moisture, as indicated. In this way, the food formula consists of fresh microalgae of the species *Chlorella vulgaris* CCAP 211/11D in the form of paste, in a proportion between 14% and 19% of the total weight of the formulation and with a degree of moisture measured at room temperature of between 81% and 86%, both inclusive.

In an even more particular case of the invention, the fresh microalgae contained in the food formula are also discoloured. In these cases, the microalgae culture has been subjected (after being harvested in the form of aqueous suspension and before forming the paste through a mechanical process of solid-liquid separation) to one or more stages of extraction or breakdown of the pigments contained in the *Chlorella,* which are in some cases undesirable in the end product (or at least in the degree to which they are contained naturally in the microalga), given that the colouring derived from the presence of microalgae in the formulation, and thus in the product in which they are incorporated, may be a drawback for the consumer's acceptance of the product. It should be indicated that the food formulation in the form of paste is pleasant from the organoleptic point of view, with no need for discolouration; nevertheless, some consumers may wish this ingredient to be recognisable in the food, but without altering too much the organoleptic properties thereof. In these cases, the particular discoloured formulation offers advantages when being introduced into the market, allowing a greater variety of consumers to be covered, depending on their tastes.

In addition, in an even more preferable manner, the microalgae (discoloured or not) in the food formulation are also deodorised. In these cases, the culture (which may also be subjected to discolouring) is also deodorised, to reduce the smell characteristic of the algae (as will be seen later, preferably by exposure to a constant flow of ozone under permanent agitation). As indicated in the previous case with regard to the colour, the natural smell of the algae may not be desirable for certain consumers (in particular if they are not accustomed to it), thus offering them the alternative of not having this aroma, or at least not so intense an aroma.

The formulation which is the object of interest, in any of its described forms, may be used as an additive or food ingredient, preferably for the manufacture of bread-making, industrial bakery and confectionery products. It is thus a food matrix which gives the product to which it is added the functional, nutritional and organoleptic qualities described above for the type of algae in question. It may thus be concluded that another object of this invention is the use as a food additive of the formulation comprised in the paste of fresh microalgae of the species *Chlorella vulgaris,* in a proportion between 10%-20% by weight of the total of the formulation, and with a degree of moisture measured at room temperature of between 80% and 90%, both inclusive, and preferably for use in industrial bread-making, bakery and confectionery products.

This invention also refers to a method of obtaining the food formula, which comprises subjecting an aqueous culture of fresh microalgae of the species *Chlorella vulgaris* to a mechanical process of solid-liquid separation to form a paste of the microalgae with a microalgae content of between 1 4%-19% by weight of the total of the formulation, and with a degree of moisture between 81%-86%. This process eliminates part of the water contained in the aqueous culture in which the microalgae are contained, to the point where a paste can be obtained.

In the most preferred case, the mechanical process of solid-liquid separation to which the culture is subjected is a centrifugation process. Preferably, said stage is carried out using a centrifuge, appropriately a high powered centrifuge. This high power centrifuge is preferably an AlfaLaval homogeniser. The time intervals used in the centrifuging depend on the concentration of microalgae measured in the aqueous culture, and this varies depending on multiple factors in the culture process. For this reason, a time must be chosen during which the centrifuge absorbs the suspension (which is the aqueous culture) with a certain flow rate, eliminating water and retaining the quantity of microalgae of interest; once this time has elapsed, depending on the concentration of the microalgae in the suspension, it is increased (if it is very diluted) or reduced (if it is very concentrated) so that the moisture in the paste obtained is finally between the intervals described above. It is not recommendable for the moisture content of the paste to be lower than that specified in this report, because the product sticks to the walls of the centrifuge container and cannot be discharged.

In practical terms, discharge times of between 480 and 640 seconds have been tested, to obtain the desired moistness. The absorption flow rate of the microalgae suspension is around 1m³/h at 9000 rpm at room temperature. For example, in proportion, 4800 litres of culture can give some 1000 litres of harvested product, and from this around 7-10 kg of algae paste.

In a particular embodiment of the invention, the proposal is to grow and harvest the microalgae which will be subjected to mechanical separation to form the paste, as this allows the properties of the culture from which they come to be monitored. In this way, said microalgae are grown in aqueous suspension until an acceptable concentration of microalgae is achieved, so that a part of the culture containing the diluted algae can be harvested. This harvest is then subjected to the mechanical separation process, preferably by centrifuging, to promote the removal of liquid and thus obtain the algae paste which will be used in the formulation of the bakery and confectionery products.

As far as the medium for growing the microalgae is concerned, the preferred medium was 3N-BBM+V, i.e. a culture Bold Basal Medium with 3-fold nitrogen and vitamins.This medium consists principally of sodium nitrate and potassium hydrogen phosphate as the majority nutrients, in addition to calcium chloride and magnesium sulphate and oligo-elements as minority components, vitamins and minerals which promote the proliferation of the culture.

In addition, the method of obtaining the food formulation may comprise a stage for discolouration of the microalgae, given the intense green colour which characterises the culture of said microalgae. The obtaining of discoloured microalgae in the fresh state (after being grown and harvested) to prepare the food formulations is an advantage of the invention in the technical field. This is particularly true because the viability of discolouration in the natural state has been recorded; in this state, the microalgae are susceptible to morphological changes and it is thus possible to vary these properties, compared to the usually commercialised lyophilised or dried algae (this being the only way of commercialising these algae with the possibility of using them to make food formulae, and for consumption they are usually obtained in the form of tablets or pills).

Although the options for discolouring the algae culture include the application of thermal stress, together with the application of different reactants for defined times or the use of mixotrophic cultures (for example, with glycerol), none has demonstrated such advantageous effects as the use of solvent acting as an oxidising agent, with the preferred option being sodium hypochlorite. It has thus been shown that algae cultures can be exposed to concentrations of this compound (for example, between 200-400 ppm depending on the concentration of the cell culture), under constant agitation at room temperature for 24hrs, thus obtaining the desired discolouration of the algae.

The action of discolouring the algae culture can be carried out either before or after the mechanical separation (centrifugation) stage, although it has been shown that the algae lose their colour in a more advantageous and preferable manner before this last stage, i.e. before the culture is subjected to centrifugation. For this reason, in a preferred case the culture of microalgae in suspension is subjected to a discolouring action before the paste is formed by centrifugation. If a solvent is used, any supernatant or possible remains of the solvent should be removed.

In addition, in a preferred manner, the method for obtaining the formulation, irrespective of whether or not a discolouring stage is included, may comprise a deodorising stage of the fresh (natural) microalgae of the formulation. In a particular embodiment, said stage may consist of subjecting the microalgae suspension or culture to a constant flow of ozone, which completely destroys the organic smells by accelerating their oxidation. In a preferred manner, the conditions in which the algae are subjected to deodorisation are 1000-1200 mg O₃/L for 12-24 hrs. Nevertheless, it is not always necessary to include the deodorisation stage, as it has been shown that the products in which these compounds can potentially be used may include aromas and flavours able to mask the slight smell which may remain after the harvesting of the microalgae. In addition, in another particular embodiment of the method which is much more preferable than the previous one because it is less aggressive on the culture, the microalgae suspension or culture is subjected to freezing before being centrifuged, at temperature conditions between -18°C and -20°C for a time of between 12-24 hours; this conserves the culture, which has its aromatic capacity reduced and remains stable over time.

Another object of this invention consists of a food product which comprises in its composition the previously defined algae-based formulation, as an additive or matrix, which gives said product the organoleptic and nutritional properties intended with the algae, provided in a natural way (fresh). Said product is preferably an industrial bread-making, bakery and confectionery product, which may be selected from the group comprising batter-based products (cupcakes, "sobao" sponges, unleavened sponges ("bizcochada"), muffins, "glorias", sweet soft cookies ("bocaditos") and other similar cakes), pastry dough-based products (puff pastries, apple tarts, "lazos", "triangulos", millefeuilles, etc.), fermented dough products (toasted bread, croissants, brioche-style bread, "ensaimada" and the like), products based on dry pastes ("tortas", almond cakes, "rosegones" and the like), those based on sheets of sponge ("bulgaros", "bracitos", "chapelas" and "cuadraditos", which are known as "Swiss roll"-type cakes), those based on special bread dough (sliced bread, burger buns and hot dog buns), and any type of cold or fried pastries (doughnuts and filled doughnuts). More preferably, the food products which comprise said algae-based formulation are selected from the group comprising: cupcakes, croissants, muffins, brioche-style bread, puff pastries, sweet soft cookies ("bocaditos") and rolls in various formats, among the most important.

The incorporation of the algae-based food formulation in the food product offers the advantages of maintaining the fundamental properties of the food, while incorporating the organoleptic and nutritional properties which define the formulation of the algae. This is possible thanks to the advantages offered by said formulation in the form of moist paste in terms of its possibility of interacting and binding with the rest of the ingredients in the bread-making, bakery and confectionery product during its preparation, thanks to the moisture present. It has also been shown that the algae-based food formulation can be fermented during the preparation of the product and maintain its capacity to retain CO₂ in the prepared dough, while also contributing the maximum quantity of bioavailable nutrients thanks to the algae. The ability to ferment and retain gas is very favourable in bread-making in that the proteins in the flour (gluten) must be arranged in the space in such a way that they can form a three-dimensional network when the dough is hydrated, so that said structure retains the CO₂ bubbles generated during the fermentation and/or during the baking process. In this way, the microalgae formulation which acts as an additive or food matrix contributes to the macromolecular framework of the dough and allows the gas produced by the yeasts during fermentation to be retained.

Preferably, for these favourable conditions to occur in a more advantageous manner, the food product contains the algae-based formulation in an amount between 0.05% and 5%, preferably between 2% and 5% of the total weight of the composition of the product. This amount will depend on the type of dough used (and required) as principal matrix. Thus, when the food product is made on the basis of batter, as in the case of cupcakes and soft cookies, the amount of the formulation is preferably between 2% and 4%, and more preferably between 2% and 3%. When the food product uses fermented dough, as in the case of the croissant, the amount of the formulation is preferably between 2% and 5%, and more preferably between 2.5% and 4%. When the food product uses special bread dough, as in the case of the burger bun, brioche-style or Fitz-style bread, the amount of the formulation is preferably between 2.5% and 4%, and more preferably between 2.5% and 3.5%. When the food product is made on the basis of toasted bread, the amount of the formulation is preferably between 2% and 4%, and more preferably between 2% and 3%. In all the intervals given here, both limits are inclusive.

When adapting the composition of the conventional food product to the incorporation of the algae-based formulation, it was considered preferable to add to the composition of the product an effective amount of a promoting agent (raising agent, yeast) owing to the great strength of the components, in order to obtain a dough with improved sponginess and honeycomb effect which can satisfy the requirements of the current market.

Thus, the food product of interest here is defined, thanks to the addition of the microalgae formulation, as containing at least 50% of its unsaturated fatty acids and at least 20% of proteins with high biological value, in which the fundamental source is the fresh paste of *Chlorella vulgaris* algae culture (with a low calorie contribution).

In this way, the preparation of the food product for bread-making, bakery and confectionery involves adding the food formulation as an additive when preparing the product dough by mixing, which may in the most preferred cases be selected from batter, fermented dough, special bread dough, or toasted bread before baking.

In a preferred case, to make the food product from the formulation which is the object of interest, it is preferable to add malt flour or caramel, which relatively reduces or masks its green colouring.

Detailed below are some particular, preferred embodiments of food products in the bread-making, bakery and confectionery industry according to this invention, which contains as an additive the formulation which is the object of interest: cupcakes, soft cookies, croissant, burger bun, brioche-style bread, Fitz-style bread and toasted bread. The more particular embodiments of these cases are illustrated (although not in a manner which limits the invention) in the examples.

Thus, most preferably, the food product comprises a base formulation comprising the following ingredients:
- Flour: 16.00 - 48.00%
- Sugar: 0.70 - 22.50%
- Water: 5.00 - 25.50%
- Salt: 0.02 - 1.15%
- Food formulation: 2% - 5%, preferably 2% - 4%,
both inclusive, and where the percentages by weight are in respect to the final weight of the product. This basic composition is shared by any of the products covered in this report.

In addition, in even more preferred cases, the products of interest may comprise (in addition to the above) the following ingredients, depending on the type of product in question:
▪ Cupcake: comprises in addition to the above
- Sunflower oil: 21.00-22.00%
- Egg white: 14.50% - 15.50%
- Egg yolk: 6.50-7.20%
- Sorbitol: 2.50-3.20%
- Tartaric acid: 0.30-0.34%
- Sodium bicarbonate: 0.65-0.75%.
- Alkaline cocoa: 2.30-2.60%
- Chocolate aroma: 0.12-0.18%

As commented earlier, in the case of cupcakes, the microalgae formulation is preferably contained in a percentage of the total weight of between 2% and 4%, and more preferably between 2% and 3%.
• Soft cookies
- Sunflower oil: 1.80-2.10%
- Egg white: 12.00-13.50%
- Egg yolk: 9.50-11.50%
- Sorbitol: 2.10-2.25%
- Powdered milk: 0.30-1.20%
- Potassium sorbate: 0.12-0.20%
- Vanillin: 0.05-0.12%
- Sodium bicarbonate: 0.40-0.70%
- Pyrophosphate: 0.40-0.70%
- Plant fibre: 4.50-5.50%
- Wholemeal bran: 4.50-5.50%
- Meliose: 0.12-0.18%
- Glycerol: 2.20-2.60%

∘ *Pear and yoghurt soft cookie:*
   - Powdered yoghurt: 0.25-0.32%
   - Yoghurt aroma: 0.15-0.25%
   - Apple-pear aroma: 0.15-0.25%
   - Fruit preparation: 2.50-3.50%
∘ *Orange soft cookie:*
   - Orange aroma: 0.12-0.17%
   - Orange juice: 1.08-1.18%
∘ *Pumpkin soft cookie:*
   - Vanilla aroma: 0.05-0.10%
   - Lemon aroma: 0.05-0.10%
   - Powdered pumpkin: 0.60-0.90%

As commented earlier, in the case of soft cookies, the formulation is preferably contained in a percentage by weight of the total of between 2% and 4%, and more preferably between 2% and 3%.
▪ Croissant
- Egg yolk: 1.80-2.20%
- Wheat gluten: 0.07-0.12%
- Whey permeate: 1.25-1.50%
- Calcium propionate: 0.11-0.16%
- Ascorbic acid: 0.008-0.012%
- Improver: 0.11-0.16%
- Guar gum: 0.07-0.12%
- Bacterial enzymes: 0.008-0.012%
- Dextrose: 2.25-2.80%
- Maltodextrin: 1.60-1.90%
- Sunflower oil: 6.70-6.95%
- Apple-pear aroma: 0.15-0.25%
- Yeast: 0.80-1.05%
- Milk protein: 1.70-2.04%
- Carboxymethylcellulose: 0.25-0.32%
- Ice: 2.05-2.40%
- Glazed apple: 9.25-9.68%
- Emulsifier: 2.60-2.85%

As commented earlier, in the case of croissants, the formulation is preferably contained in a percentage by weight of the total of between 2% and 5%, and more preferably between 2.5% and 4%.
▪ Special burger and chia buns
- Ascorbic acid: 0.02-0.05%
- Liquid sourdough: 18.45-18.70%
- Wheat gluten: 1.30-1.55%
- Calcium propionate: 0.08-0.15%
- Improver 1: 1.05-1.18%
- Improver 2: 0.08-0.14%
- Emulsifier: 0.15-0.25%
- Bacterial enzymes: 0.025-0.038%
- Vitalbakery enzymes: 0.008-0.012%
- Fibre: 1.30-1.45%
- Malt flour: 0.015-0.028%
- Olive oil: 2.05-2.20%
- Sodium diacetate: 0.006-0.012%
- Whey permeate: 2.35-2.55%
- Bread aroma: 0.08-0.14%
- Inactive yeast: 0.08-0.14%
- Encapsulated sorbic acid: 0.12-0.17%
- Chia seeds: 8.20-8.75%

In the case of special bread, the formulation is preferably contained in a percentage by weight of the total of between 2.5% and 4%, and more preferably between 2.5% and 3.5%.
▪ Special Fitz-style bread
- Olive oil: 1.90-2.10%
- Wheat gluten: 1.00-1.35%
- Xanthan gum: 0.05-0.12%
- Improver: 0.55-0.70%
- Ascorbic acid: 0.008-0.012%
- Calcium propionate: 0.08-0.20%
- Emulsifier: 0.18-0.30%
- Encapsulated sorbic acid: 0.15-0.25%
- Corn starch: 0.95-1.20%
- Fibre: 1.50-1.85%
- Maltodextrin: 0.95-1.25%
- Garlic: 0.70-0.95%
- Ice: 7.50-9.00%

In the case of special bread, the formulation is preferably contained in a percentage by weight of the total of between 2.5% and 4%, and more preferably between 2.5% and 3.5%.
▪ Brioche-style bread
- Egg white: 5.40-5.80%
- Egg yolk: 2.35-2.55%
- Wheat gluten: 0.85-1.15%
- Powdered milk: 1.05-1.35%
- Vanillin: 0.07-0.12%
- Calcium propionate: 0.10-0.16%
- Ascorbic acid: 0.008-0.012%
- Improver: 0.85-1.15%
- Sunflower oil: 6.35-6.80%
- Milk aroma: 0.12-0.18%
- Butter aroma: 0.12-0.16%
- Beta-carotene: 0.008-0.012%
- Yeast: 0.55-0.75%
- Lactic acid: 0.06-0.09%
- Carboxymethylcellulose: 0.22-0.28%
- Ice: 2.95-3.15%
- Fruit: 2.20-2.75%
- Omega 3: 3.00-3.45%

In the case of brioche-style bread, the formulation is preferably contained in a percentage by weight of the total of between 2.5% and 4%, and more preferably between 2.5% and 3.5%.
▪ Toasted bread
- Palm oil: 5.7-5.95%
- Yeast: 5.25-5.52%
- Malt extract: 0.75-1.05%
- Improver: 0.22-0.29%
- Ascorbic acid: 0.01-0.03%
- Cereal mix: 5.58-5.95%

In the case of toasted bread, the formulation is preferably contained in a percentage by weight of the total of between 2% and 4%, and more preferably between 2% and 3%.

### Examples

### Example 1. Nutritional features of the strains of Chlorella vulgaris which is the subject of this invention as a principal component of the food formulation.

The nutritional characteristics of the preferred strains used to obtain the fresh paste were given, in terms of the amount of protein and fatty acid profile, using conventional laboratory techniques.

### - Protein determination:

To determine the amount of protein present in the *Chlorella vulgaris* cells, the cell protein was extracted by boiling the cells for 5 minutes with NaOH 1M. The protein extracted was determined using the bicinchoninic acid or BCA kit. Prior to this, the corresponding dilution was applied to the samples so that the concentration of NaOH was 0.25M. The absorbance values obtained were interpolated on a BSA straight-line pattern in NaOH 0.25M. The protein quantification results are shown in Table 1.

**Table 1. Amount of protein in Chlorella vulgaris cells**

| ***BSA (500 µg*/*ml)*** | ***Protein (µg*/*ml)*** |
|---|---|
| 211/12 | 31.91 ± 0.45 |
| 211/19 | 34.88 ± 1.77 |
| 211/11B | 37.40 ± 4.38 |
| 211/11D | 36.08 ± 1.03 |
| 211/52 | 35.30 ± 2.63 |

It can be seen from the results that all the strains of *Chlorella vulgaris* analysed contain very similar protein values and are around 30%.

### - Fatty acid profile

To determine the fatty acid profile in *Chlorella vulgaris* cells, the membrane lipids were extracted using chloroform from a pellet of cells of each of the strains to be analysed. The fatty acids extracted were subjected to a process of methanolysis, giving rise to the methyl esters of the fatty acids, which were detected and identified using a gas chromatograph coupled to a mass detector. On the one hand, cultures grown in flasks were analysed, and on the other, cultures grown in a fermenter. The results of the lipid analysis are shown in Tables 2 and 3.

**Table 2. Profile of fatty acids in Chlorella vulgaris cells (in a flask)**

| ***211*/*11D*** | ***211*/*19*** | ***211*/*52*** | ***211*/*11B*** | ***211*/*12*** |
|---|---|---|---|---|
| - | Linoleic Acid (18:2) | Linoleic Acid (18:2) | Linoleic Acid (18:2) | Linoleic Acid (18:2) |
| - | α-Linolenic acid (18:3) | α-Linolenic acid (18:3) | α-Linolenic acid (18:3) | α-Linolenic acid (18:3) |
| 6,9-octadecadienoic acid | - | - | - | - |
| γ-Linolenic acid (18:3) | - | - | - | - |
| - | - | Palmitic acid (16:0) | Palmitic acid (16:0) 0) | - |
| 14-Methyl-pentadecanoic acid | 14-Methyl-pentadecanoi c acid | - | - | 14-Methyl-pentadecanoi c acid |
| - | - | 7,10-hexadecanoic acid | 7,10-hexadecanoic acid | - |
| - | - | - | 7,10-eicosatrenoic acid | - |

**Table 3. Profile of fatty acids in Chlorella vulgaris cells (in a fermenter)**

| ***211*/*11D*** | ***211*/*19*** | ***211*/*52*** | ***211*/*11B*** |
|---|---|---|---|
| Palmitic acid (16:0) | Palmitic acid (16:0) | Palmitic acid (16:0) | Palmitic acid (16:0) |
| Linoleic Acid (18:2) | Linoleic Acid (18:2) | Linoleic Acid (18:2) | Linoleic Acid (18:2) |
| Oleic Acid (18:1) | - | - | - |
| - | α-Linolenic acid (18:3) | - | α-Linolenic acid (18:3) |
| - | - | γ-Linolenic acid (18:3) | - |
| - | - | 6,9,12,15-docosatetraenoic acid | 6,9,12,15-docosatetraenoic acid |
| Myristic acid (14:0) | - | - | - |
| - | Hexadecatrienoic acid | - | - |
| Stearic acid (18:0) | - | - | Stearic acid (18:0) |
| - | - | 14,17-Octadecadienoic acid | - |
| - | - | 7,10-hexadecadienoic acid | - |
| - | - | - | 9,12-hexadecadienoic acid |
| Eicosanoic acid | - | - | - |
| Docosaonioc acid | - | - | - |
| Tetracosanoic acid | - | - | - |
| 14,17-octadecad ienoic acid | - | - | - |

Different fatty acids were identified in the cultures of the five strains of *Chlorella vulgaris* analysed. Some of them coincide for the different strains, while others are identified only in one or more of them, which gives examples of the versatility of the formulation to be prepared from them. Differences between the results obtained from cultures in a flask and in a fermenter for the same strain are also seen. Given the size of the peaks observed in the chromatographs, it is possible that only the majority fatty acids have been identified in each sample.

It can be concluded that the protein content determined was similar for all of them, whereas the composition in fatty acids depends on the growth conditions for the culture and for the strain considered.

### Example 2. - Study of the effect of the food formulation which is the object of the invention on flour for industrial bread-making, bakery and confectionery products.

A flour commonly used in the preparation of bread-making, bakery and confectionery products was added with a specific amount of 0.7% of several of the strains of *Chlorella vulgaris* selected as preferred: 211/19, 211/52 and 211/11D. These flours were compared with a sample of the flour with no additions and in terms of:
- Quantity of protein
- Quantity of fat
- Amino acid profile
- Fatty acid profile
- Quantity of DHA (docosahexaenoic).

The results for quantity of protein and amino acid profile of the flour with the three strains of *Chlorella vulgaris* added are shown in Table 4. In addition, the results for quantity of fat and fatty acid profile are shown in Table 5.

On the basis of the results, small differences are observed in terms of the amino acid profile of the three strains analysed, compared to each other and to the control flour, which could be due in part to the low concentration of the strain. However, these small differences are very interesting in the case of the fatty acid profile. On the one hand, we can see that the cis-linoleic acid (omega-3) is increased in the flour with the different strains of *Chlorella vulgaris* added, and the effect is greatest in the case of the 211/11D strain. On the other hand, the docosahexaenoic acid (omega-3) is not detected in the flour, but it is detected in the flour with the different strains of C. *vulgaris* added. Finally, the cysteine acid is increased by the addition of any of the three strains studied in the flour. These results suggested that the different strains of *Chlorella vulgaris* analysed have a great potential in terms of their polyunsaturated omega-3 fatty acid content.

**Table 4. Quantity of protein and amino acid profile of the flour and of the flour with three of the selected Chlorella vulgaris strains added**

| ***Analysis*** | ***Flour*** | ***Flour* + *211*/*19 (0.7%)*** | ***Flour* + *211*/*11D (0.7%)*** | ***Flour* + *211*/*52 (0.7%)*** |
|---|---|---|---|---|
| Total protein (g/100g) | 11.30 | 11.69 | 11.72 | 11.76 |
| Leucine* (%) | 0.82 | 0.84 | 0.80 | 0.84 |
| Isoleucine* (%) | 3.34 | 0.32 | 0.30 | 0.32 |
| Lysine* (%) | 0.22 | 0.23 | 0.23 | 0.23 |
| Methionine* (%) | 0.15 | 0.13 | 0.17 | 0.14 |
| Phenylalanine* (%) | 0.63 | 0.68 | 0.66 | 0.69 |
| Threonine* (%) | 0.35 | 0.36 | 0.34 | 0.37 |
| Tryptophan* (%) | 0.09 | <0.05 | <0.05 | <0.05 |
| Valine* (%) | 0.49 | 0.42 | 0.41 | 0.43 |
| Histidine (%) | 0.26 | 0.21 | 0.21 | 0.20 |
| Glycine (%) | 0.45 | 0.46 | 0.47 | 0.46 |
| Glutamic Acid (%) | 4.36 | 4.43 | 4.39 | 4.38 |
| Hydroxyproline* (%) | <0.05 | <0.05 | <0.05 | 0.09 |
| Proline (%) | 1.63 | 0.61 | 1.57 | 1.58 |
| Alanine (%) | 0.38 | 0.39 | 0.39 | 0.39 |
| Arginine (%) | 0.44 | 0.45 | 0.44 | 0.44 |
| Tyrosine (%) | 0.27 | 0.20 | 0.20 | 0.19 |
| Cysteine (%) | 0.26 | 0.36 | 0.41 | 0.36 |
| Serine (%) | 0.67 | 0.62 | 0.62 | 0.63 |
| Aspartic acid (%) | 0.57 | 0.49 | 0.51 | 0.50 |

| | | | | |
|---|---|---|---|---|
| (*) Essential amino acids | | | | |

**Table 5. Quantity of fat and fatty acid profile of the flour and of the flour with three of the selected Chlorella vulgaris strains added**

| ***Analysis*** | ***Structure*** | ***Flour*** | ***Flour* + *211*/*19 (0.7%)*** | ***Flour* + *211*/*11D (0.7%)*** | ***Flour* + *211*/*52 (0.7%)*** |
|---|---|---|---|---|---|
| Total fat (g/100g) | | 1.60 | 1.76 | 1.53 | 1.68 |
| Caprylic acid (%) | C8:0 | 0.00 | 0.00 | 0.00 | 0.00 |
| Capric acid (%) | C10:0 | 0.00 | 0.00 | 0.00 | 0.00 |
| Lauric acid (%) | C12:0 | 0.00 | 0.00 | 0.00 | 0.00 |
| Myristic acid (%) | C14:0 | 0.27 | 0.06 | 0.10 | 0.09 |
| Palmitic acid (%) | C16:0 | 16.93 | 16.22 | 16.33 | 16.34 |
| Palmitoleic acid (%) | C16:1 | 0.00 | 0.17 | 0.17 | 0.15 |
| Stearic acid (%) | C18:0 | 0.82 | 1.01 | 1.06 | 1.46 |
| Trans-oleic acid (%) | C18:1 | 0.00 | 0.00 | 0.00 | 0.00 |
| Cis-oleic acid (%) | C18:1 | 14.33 | 12.82 | 12.57 | 12.77 |
| Cis-linoleic acid (%) | C18:2 | 63.45 | 62.23 | 62.30 | 61.94 |
| Trans-linoleic acid (%) | C18:2 | 0.00 | 0.09 | 0.09 | 0.07 |
| Cis-linolenic acid (%) | C18:3 | 3.71 | 4.88 | 5.63 | 4.76 |
| Trans-linolenic acid (%) | C18:3 | 0.00 | 0.00 | 0.00 | 0.00 |
| Arachidonic acid (%) | C20:4 | 0.00 | 0.00 | 0.00 | 0.00 |
| Docosahexaenoic acid DHA (%) | C22:6 | 0.00 | 0.29 | 0.31 | 0.25 |
| Trans fatty acids (%) | | 0.00 | 0.09 | 0.09 | 0.07 |

In conclusion, the results of the comparison between a normal flour and the same flour with the different strains at 0.7% added in the laboratory suggested that the amino acid profile does not vary significantly between them, and the different strains of *Chlorella vulgaris* analysed have great potential in terms of their polyunsaturated omega-3 fatty acids content.

### Example 3.- Preparation of typical food products in the bread-makinq, bakery and confectionery industry from the formulation in the invention.

### 3.1.- Addition of the formulation in batter-type dough for the preparation of cupcakes

A batter dough for cupcakes was prepared using traditional methods, incorporating in the process as an additive or matrix the formulation which is the object of interest, with the strain 211/11D and 211/52 with a degree of moisture of approximately 83%. The ingredients, correctly weighed, were placed in the hand or automatic beater, depending on the raw material considered. The agitation process was around 3 minutes at room temperature. Once the paste was homogeneous, the dosing stage was carried out, with the quantity agreed beforehand deposited in different individual capsules which were led through the oven for the appropriate time to allow the dough/batter to be promoted and then cooked, to remain at rest for the necessary time in the cooling stage.

To adjust the mixture in such a way that the fundamental properties of the batter typical of these products were maintained, the amount of promoting agent (tartaric acid and sodium bicarbonate) and flour was increased slightly to prevent the dough/batter from sticking, and the fat content was reduced to adjust the texture and the sponginess, obtaining the compositions in the table.

**Table 6. Composition in % by weight of the total dough for cupcakes prepared from the batter containing the algae formulation which is the object of the invention**

| ***Ingredients*** | ***Cupcake type 1*** |
|---|---|
| Flour | 25.22% |
| Sugar | 22.39% |
| Sunflower oil | 21.47% |
| Egg white | 15.08% |
| Egg yolk | 7.08% |
| Sorbitol | 3.06% |
| Tartaric acid | 0.36% |
| Sodium bicarbonate | 0.69% |
| Salt | 0.05% |
| Alkaline cocoa | 2.42% |
| Chocolate aroma | 0.14% |
| ***Algae formulation (moisture 83%)*** | 2.06% |

### 3.2.- Addition of the formulation in batter for the preparation of soft cookies (pear and yoghurt, orange or pumpkin)

A batter dough was prepared using traditional methods, incorporating in the process as an additive or matrix the formulation which is the subject of interest, with the strain ***211*/*11D*** and 211/52 with a degree of moisture of approximately 83%. The ingredients, correctly weighed, were placed in the hand or automatic beater, depending on the raw material considered. The agitation process was around 3 minutes at room temperature. Once the paste was homogeneous, the dosing stage was carried out, with the quantity agreed beforehand deposited in individual moulds which were led through the oven for the appropriate time to allow the dough/batter to be promoted and then cooked, to remain at rest for the necessary time in the cooling stage. The cutting and packaging stage was then carried out.

**Table 7. Composition in % by weight of the total dough for soft cookies prepared from the batter containing the algae formulation which is the object of the invention**

| ***Ingredients*** | ***Pear and yoghurt*** | ***Orange*** | ***Pumpkin*** |
|---|---|---|---|
| Flour | 17.75% | 18.27% | 18.17% |
| Sugar | 19.23% | 19.79% | 19.69% |
| Sunflower oil | 1.97% | 2.03% | 2.02% |
| Soya oil | 12.33% | 12.68% | 12.62% |
| Egg white | 12.33% | 12.68% | 12.62% |
| Egg yolk | 9.86% | 11.16% | 11.11% |
| Sorbitol | 2.12% | 2.18% | 2.17% |
| Water | 4.44% | 5.07% | 5.05% |
| Powdered milk | 0.49% | 0.66% | 1.16% |
| Salt | 0.02% | 0.02% | 0.02% |
| Potassium sorbate | 0.17% | 0.15% | 0.17% |
| Vanillin | 0.09% | - | 0.08% |
| Sodium bicarbonate | 0.47% | 0.53% | 0.63% |
| Pyrophosphate | 0.47% | 0.53% | 0.53% |
| Plant fibre | 4.93% | 5.07% | 5.05 % |
| Wholemeal bran | 4.93% | 5.07% | 5.05% |
| Meliose | 0.17% | - | 0.15% |
| Glycerol | 2.37% | 2.44% | 2.42% |
| Powdered yoghurt | 0.29% | - | - |
| ***Algae formulation (moisture 83%)*** | 2.22% | 2.30% | 2.23% |
| Yoghurt aroma | 0.21% | - | - |
| Apple-pear aroma | 0.20% | - | - |
| Fruit preparation | 2.96% | - | - |
| Orange aroma | - | 0.15% | - |
| Orange juice | - | 1.12% | - |
| Vanilla aroma | - | - | 0.08% |
| Lemon aroma | - | - | 0.08% |
| Powdered pumpkin | - | - | 0.76% |

### 3.3.- Addition of the formulation in fermented type dough for the preparation of croissants

A fermented dough for croissants was prepared using traditional methods, incorporating in the process as an additive or matrix the formulation which is the object of interest, with the strain 211/11D and 211/52 with a degree of moisture of approximately 83%. Once the different ingredients which would form part of the food matrix had been kneaded together, the product was allowed to rest so that the bacteria and yeasts could carry out the fermentation process (3 hours and 30 min). Subsequently, the cooking stage was carried out, during which the microorganisms were made inactive and the product was completed. After cooling, it was ready for packaging.

In this case, the quantity of the algae-based formulation was increased to prevent in this case a slight colouring of the dough, reducing in turn the amount of flour, because of increased sticking of the dough. The yeast content was also increased to improve the fermentation. The end product exhibits improved organoleptic properties, with only a slight smell of algae and a pleasant taste. The texture is very similar to that of an original croissant.

**Table 8. Composition in % by weight of the total croissant dough prepared from the fermented dough containing the algae formulation which is the object of the invention**

| ***Ingredients*** | ***Croissant*** |
|---|---|
| Flour | 48.60% |
| Sugar | 5.48% |
| Egg yolk | 2.05% |
| Water | 15.06% |
| Wheat gluten | 0.09% |
| Whey permeate | 1.37% |
| Calcium propionate | 0.14% |
| Ascorbic acid | 0.01% |
| Improver | 0.14% |
| Guar gum | 0.09% |
| Bacterial enzymes | 0.01% |
| Dextrose | 2.51% |
| Maltodextrin | 1.83% |
| Sunflower oil | 6.84% |
| Salt | 0.46% |
| Apple-pear aroma | 0.23% |
| ***Algae formulation (moisture 83%)*** | 0.46% |
| Yeast | 0.91% |
| Milk protein | 1.83% |
| Carboxymethylcellulose | 0.29% |
| Ice | 2.28% |
| Glazed apple | 9.58% |
| Emulsifier | 2.74% |

### 3.4. Addition of the formulation to special dough for the preparation of burger and chia buns

A special dough for burger and chia buns was prepared using traditional methods, incorporating in the process as an additive or matrix the formulation which is the object of interest, with the strain 211/11D and 211/52 with a degree of moisture of approximately 83%. After homogenisation of the dough and kneading of all the ingredients, it was divided into individual units and made into balls to be placed in the mould. After approximately 3 hours in the fermentation tower, the product was baked and then cooled as the final stage.

Using the conventional formulation as a base, the quantity of yeast was increased slightly and the flour content readjusted to soften the dough and reduce its strength, in turn reducing the water contribution and thus increasing the algae formulation content. In one variant, powdered tomato was incorporated as a new ingredient, to try out possible combinations of colours which could be used. The product described in the table in both its variants exhibited ideal flavour and texture conditions, attractive to look at, with a honeycomb effect similar to conventional special bread. This fact demonstrates that the incorporation of the algae formulation does not affect the wheat flour's ability to retain the gas.

**Table 9. Composition in % by weight of the total burger and chia bun dough prepared from the special dough containing the algae formulation which is the object of the invention**

| ***Ingredients*** | ***Burger and chia bun*** |
|---|---|
| *Flour* | 32.74% |
| Sugar | 5.17% |
| Water | 19.53% |
| Ascorbic acid | 0.03% |
| Liquid sourdough | 18.96% |
| Wheat gluten | 1.44% |
| Calcium propionate | 0.11% |
| Improver 1 | 1.15% |
| Improver 2 | 0.11% |
| Emulsifier | 0.20% |
| Bacterial enzymes | 0.03% |
| Vitalbakery enzymes | 0.01% |
| Plant fibre | 1.38% |
| Malt flour | 0.02% |
| Olive oil | 2.14% |
| Salt | 0.56% |
| Sodium diacetate | 0.01% |
| Whey permeate | 2.48% |
| Bread aroma | 0.11% |
| ***Algae formulation (moisture 90%)*** | 2.54% |
| Inactive yeast | 0.11% |
| Encapsulated sorbic acid | 0.14% |
| Hercules yeast | 2.26% |
| Chia seeds | 8.47% |

### 3.5. - Addition of the formulation in special Fitz-style breads

A special bread dough was prepared using traditional methods, incorporating in the process as an additive or matrix the formulation which is the object of interest, with the strain 211/11D and 211/52 with a degree of moisture of approximately 83%. After homogenisation of the dough and kneading of all the ingredients, the dough is flattened out and allowed to ferment for approximately 3 hours 10 min. Once this time had elapsed, the product was baked, cut, cooled and packaged as the final stage.

**Table 10. Composition in % by weight of the total dough for Fitz-style bread prepared from dough containing the algae formulation which is the object of the invention**

| ***Ingredients*** | ***Fitz*** |
|---|---|
| *Flour* 1 | 7.42% |
| *Flour* 2 | 34.04% |
| Sugar | 5.81% |
| Olive oil | 2.03% |
| Salt | 0.90% |
| Water | 22.59% |
| Wheat gluten | 1.10% |
| Xanthan gum | 0.08% |
| Improver | 0.65% |
| Ascorbic acid | 0.01% |
| Calcium propionate | 0.15% |
| Emulsifier | 0.24% |
| Encapsulated sorbic acid | 0.18% |
| Corn starch | 1.10% |
| Fibre | 1.77% |
| Maltodextrin | 1.10% |
| Garlic | 0.81% |
| ***Algae formulation (moisture 83%)*** | 2.82% |
| Ice | 8.07% |

### 3.6.- Addition of the formulation in special breads such as brioche-type bread

A special dough for brioche-type bread was prepared using traditional methods, incorporating in the process as an additive or matrix the formulation which is the object of interest, with the strain 211/11D and 211/52 with a degree of moisture of approximately 83%. After homogenisation of the dough, kneading and laying in sheets, the product is then formed. After subsequent placing on the pans, the product is then fermented and finally baked.

**Table 11. Composition in % by weight of the total for brioche-style bread prepared from dough containing the algae formulation which is the object of the invention**

| ***Ingredients*** | ***Brioche-style bread*** |
|---|---|
| Flour | 48.57% |
| Sugar | 6.51% |
| Egg white | 5.64% |
| Egg yolk | 2.43% |
| Water | 15.87% |
| Gluten | 1.00% |
| Powdered milk | 1.22% |
| Vanillin | 0.09% |
| Calcium propionate | 0.14% |
| Ascorbic acid | 0.01% |
| Improver | 1.00% |
| Sunflower oil | 6.51% |
| Salt | 0.57% |
| Milk aroma | 0.14% |
| Butter aroma | 0.14% |
| Beta-carotene | 0.01% |
| Yeast | 0.60% |
| Lactic acid | 0.08% |
| Carboxymethylcellulose | 0.24% |
| Ice | 3.04% |
| ***Algae formulation (moisture 83%)*** | 2.82% |
| Fruit | 2.47% |
| Omega 3 | 3.25% |

### 3.7. - Addition of the toasted bread formulation

A bread dough was prepared using traditional methods, incorporating in the process as an additive or matrix the formulation which is the object of interest, with the strain 211/11D and 211/52 with a degree of moisture of approximately 83%. After homogenisation of the dough, kneading and forming into balls, the product is then formed. After the necessary fermentation, the product is baked for a first time, then cut, and finally toasted.

**Table 12. Composition in % by weight of the total for toasted bread prepared from dough containing the algae formulation which is the object of the invention**

| ***Ingredients*** | ***Toasted bread*** |
|---|---|
| Flour 1 | 39.50% |
| Flour 2 | 13.16% |
| Palm oil | 5.80% |
| Water | 25.14% |
| Yeast | 5.48% |
| Malt extract | 0.91% |
| Sugar | 0.78% |
| Salt | 0.91% |
| Improver | 0.26% |
| ***Algae formulation (moisture 83%)*** | 2.29% |
| Ascorbic acid | 0.02% |
| Cereal mix | 5.71% |

### Example 4.- Study of the functional properties of the food formulation which is the object of the invention in human fibroblasts and young zebra fish (at systemic level).

As regards the functional properties of the microalgae-based formulation which is the object of this invention, studies of said functionality on different systems, such as human fibroblasts and young zebra fish (at systemic level), were carried out in order to determine the signalling routes and genes regulated by the treatment with microalgae.

After the trials, the analysis of differential gene expression provided proof that the formulation which is the object of interest, seen as a microalgae dietary supplement, can benefit numerous metabolic routes. Principally, because of their medical importance, it is worth emphasising at systemic level the induction of insulin synthesis routes, melanogenesis, the repair of DNA, metabolism of starch and sugars, the metabolism of retinol, the metabolism of arachidonic acid, glycolysis/glycogenesis, apoptosis and energy metabolism. The first analysis of routes focussed principally on the insulin route, monitoring of sugar (Glucose) metabolism, given the nature of the food products covered by this invention. The data indicated that the insulin route was over-activated, in such a way that consumption of the formulation reduced the level of glucose in blood.

Meanwhile, the study of the effects of the treatment of human fibroblasts with microalgae extract showed an activation of routes related to cell survival, which evidenced its cell antiaging properties. However, the most noteworthy property of those detected in this study consisted of strong activation of routes related to the immunological system of fibroblasts. It should be borne in mind that the fibroblasts are not the cells which normally act on an immunological level, which is why it is notable that the activation of these genes was so strong.

One of the principal conclusions which can be extracted from these trials is that, in light of the physiological effects determined in vivo, there is bioavailability of the components of the *Chorella vulgaris* alga when they are ingested as part of the food formulation which is the object of this invention.

## Claims

1. A food formulation **characterised in that** it comprises fresh microalgae in the form of paste of the species *Chlorella vulgaris* in a proportion comprised between 14% and 19% of the total weight of the formulation and with an average degree of moisture at room temperature of between 81% and 86%, both inclusive, and **in that** the microalgae of the species *Chlorella vulgaris* are of the strain *Chlorella vulgaris fo. tertia* with deposit number CCAP 211/11D.

2. The food formulation according to claim 1, which consists of a paste of fresh microalgae of the species *Chlorella vulgaris,* in a proportion comprised between 14% and 19% of the total weight of the formulation and with an average degree of moisture at room temperature of between 81% and 86%, both inclusive, and wherein the microalgae of the species *Chlorella vulgaris* are of the strain *Chlorella vulgaris fo. tertia* with deposit number CCAP 211/11D.

3. The food formulation according to any one of clams 1 or 2, wherein the fresh microalgae are discoloured and deodorised.

4. A method of obtaining the food formulation described in any one of claims 1 to 3, **characterised in that** it comprises subjecting an aqueous culture of fresh microalgae of the species *Chlorella vulgaris* to a mechanical solid-liquid separation to form a paste of the microalgae with a microalgae content of between 14%-19% of the total weight of the formulation and with a degree of moisture of between 81%-86%, both inclusive, and wherein the microalgae of the species *Chlorella vulgaris* are of the strain *Chlorella vulgaris fo. tertia* with deposit number CCAP 211/11D.

5. The method according to the previous claim, wherein the mechanical solid-liquid separation is carried out by centrifugation.

6. The method according to any one of claims 4 or 5, which comprises cultivating and harvesting the microalgae which are subjected to mechanical separation to form the paste.

7. The method according to any one of claims 4 to 6, which comprises discolouring and/or deodorising the microalgae.

8. The method according to the previous claim, wherein the deodorisation is carried out by freezing the culture before subjecting it to mechanical solid-liquid separation, at a temperature of between -18°C and -20°C for a time of between 12 and 24 hours, both inclusive.

9. A food product **characterised in that** it comprises in its composition the formulation described in any one of claims 1 to 3.

10. The food product according to the previous claim, which is selected from the group consisting of bread-making products, bakery products and confectionery products.

11. The food product according to any one of claims 9 or 10, which comprises the formulation in a percentage of between 0.05% and 5% by weight of the total of the product composition.

12. The food product according to any one of claims 10 or 11, which is selected from the group consisting of products based on batter, based on pastry dough, based on fermented dough, based on dry pastes, based on sheets of sponge and based on special bread dough and fried bakery products.

13. The food product according to the previous claim, wherein the products based on batter are selected from the group consisting of cupcakes, "sobao" sponges, unleavened sponges, muffins, "gloria" and sweet soft cookies; the products based on pastry dough are selected from the group consisting of puff pastries, apple tarts, "lazo", "triangulo" and millefeuilles; the products based on fermented dough are selected from the group consisting of toasted bread, croissants, "pandorino", brioche-style bread and "ensaimada"; the products based on dry pastes are selected from the group consisting of "tortas", almond cakes and "rosegón"; the products based on a sheet of sponge are selected from the group consisting of "Swiss-roll" type cakes; the products based on special bread dough are selected from the group consisting of sliced bread, burger buns and hot dog buns; and the fried pastries are selected from the group consisting of doughnuts and filled doughnuts.

14. The food product according to any one of claims 12 or 13, wherein when said food product contains a base of batter, the amount of the formulation is between 2% and 4% by weight of the total of the product composition, both inclusive; when the food product contains a base of fermented dough, the amount of the formulation is between 2% and 5% by weight of the total of the product composition, both inclusive; when the food product contains a base of toasted bread, the amount of the formulation is between 2% and 4% by weight of the total of the product composition, both inclusive; and when the food product contains a base of special bread dough, the amount of the formulation is between 2.5% and 4% by weight of the total of the product composition, both inclusive.

15. Use of the food formulation described in any one of claims 1 to 3 as a food additive.

## Patentansprüche

1. Nahrungsmittelzubereitungsformulierungsformulierung, **dadurch gekennzeichnet, dass** sie frische Mikroalgen in Teigform der Gattung *Chlorella vulgaris* aufweist mit einem Anteil, der zwischen 14 % und 19 % des Gesamtgewichts der Zubereitungsformulierung liegt, und mit einem mittleren Anteil an Feuchtigkeit bei Raumtemperatur zwischen 81 % und 86 %, jeweils einschließlich der Grenzen, und dass die Mikroalgen der Gattung *Chlorella vulgaris* vom Stamm *Chlorella vulgaris fo. tertia* mit der Depotnummer CCAP 211/11G sind.

2. Nahrungsmittelzubereitungsformulierungsformulierung nach Anspruch 1, die aus einem Teig aus frischen Mikroalgen der Gattung *Chlorella vulgaris* besteht mit einem Anteil, der zwischen 14 % und 19 % des Gesamtgewichts der Zubereitungsformulierung liegt, und mit einem mittleren Grad an Feuchtigkeit bei Raumtemperatur zwischen 81 % und 86 %, jeweils einschließlich der Grenzen, und wobei die Mikroalgen der Gattung *Chlorella vulgaris* vom Stamm *Chlorella vulgaris* fo. Tertia mit der Depotnummer CCAP 211/11D sind.

3. Nahrungsmittelzubereitungsformulierung nach Anspruch 1 oder 2, wobei die frischen Mikroalgen gefärbt und geruchsneutralisiert sind.

4. Verfahren zum Erhalten der Nahrungsmittelzubereitungsformulierung gemäß einem der Ansprüche 1 bis 3, **dadurch gekennzeichnet, dass** das Verfahren umfasst: Unterziehen einer wässrigen Kultur aus frischen Mikroalgen der Gattung *Chlorella vulgaris* einer mechanischen Fest-Flüssig-Abscheidung zur Herstellung eines Teigs der Mikroalgen mit einem Mikroalgenanteil zwischen 14 %-19 % des Gesamtgewichts der Zubereitungsformulierung und mit einem Grad an Feuchtigkeit zwischen 81 %-86 %, jeweils einschließlich der Grenzen, und wobei die Mikroalgen der Gattung *Chlorella vulgaris* vom Stamm *Chlorella vulgaris fo. tertia* mit der Depotnummer CCAP 211/11D sind.

5. Verfahren nach dem vorhergehenden Anspruch, wobei die mechanische Fest-Flüssig-Abscheidung durch Zentrifugieren ausgeführt wird.

6. Verfahren nach Anspruch 4 oder 5, das umfasst: Kultivieren und Ernten der Mikroalgen, die der mechanischen Abscheidung unterzogen werden, um den Teig zu erzeugen.

7. Verfahren nach einem der Ansprüche 4 bis 6, das Verfärben der Mikroalgen und/oder Ausführen einer Geruchsbeseitigung an den Mikroalgen umfasst.

8. Verfahren nach dem vorhergehenden Anspruch, wobei die Geruchsbeseitigung ausgeführt wird, indem die Kultur vor ihrer mechanischen Fest-Flüssig-Abscheidung bei einer Temperatur zwischen -18 °C und -20 °C für eine Zeitdauer zwischen 12 und 24 Stunden, einschließlich der Grenzen, gefroren wird.

9. Nahrungsmittelprodukt, **dadurch gekennzeichnet, dass** es in seiner Zusammensetzung die Zubereitungsformulierung nach einem der Ansprüche 1 bis 3 enthält.

10. Nahrungsmittelprodukt nach dem vorhergehenden Anspruch, die ausgewählt ist aus der Gruppe: brotbildende Produkte, Bäckereiprodukte und Konditoreiprodukte.

11. Nahrungsmittelprodukt nach Anspruch 9 oder 10, das die Zubereitungsformulierung mit einem Anteil zwischen 0,05 % und 5 % bezogen auf das Gewicht der gesamten Produktzusammensetzung umfasst.

12. Nahrungsmittelprodukt nach Anspruch 10 oder 11, das ausgewählt ist aus der Gruppe: Produkte auf Basis von Backteig, auf Basis von Gebäckteig, auf Basis von segmentiertem Teig, auf Basis von Trockenteigen, auf Basis von Lagen aus Biskuitteig und auf Basis eines speziellen Brotteigs und frittierten Bäckereiprodukten.

13. Nahrungsmittelprodukt nach dem vorhergehenden Anspruch, wobei die Produkte auf Basis von Backteig ausgewählt sind aus der Gruppe: muffinartige Kuchen, "Sobao-" Biskuitteigen, angesäuerten Biskuitteigen, Muffins, "Gloria" und süßen Weichkeksen; die Produkte auf Basis von Gebäckteig ausgewählt sind aus der Gruppe: Blätterteig, Apfeltorten, "Lazo", "Triángulo'" und "Millefeuilles"; die Produkte auf Basis von segmentierten Teig ausgewählt sind aus der Gruppe: geröstetes Brot, Croissants, "Pandorino", Hefebrot und "Ensaimada"; die Produkte auf Basis von Trocketeigen ausgewählt sind aus der Gruppe: "Tortas", Mandelkuchen und "Rosegön"; die Produkte auf Basis von Lagen aus Biskuitteig ausgewählt sind aus der Gruppe: Kuchen als "Schweizerrollen"; die Produkte auf Basis von speziellem Brotteig ausgewählt sind aus der Gruppe: Schnittbrot, Burger-Brötchen und Hot-Dog-Brötchen; und die frittierten Gebäckwaren ausgewählt sind aus der Gruppe: Krapfen und gefüllte Krapfen.

14. Nahrungsmittelprodukt nach Anspruch 12 oder 13, wobei, wenn das Nahrungsmittelprodukt eine Basis aus Backteig aufweist, der Anteil der Zubereitungsformulierung zwischen 2 % und 4 % bezogen auf das Gewicht der gesamten Produktzusammensetzung, einschließlich der Grenzen, liegt; wenn das Nahrungsmittelprodukt eine Basis aus fermentiertem Teig enthält, der Anteil der Zubereitungsformulierung zwischen 2 % und 5 % bezogen auf das Gewicht der gesamten Produktzusammensetzung, einschließlich der Grenzen, liegt; wenn das Nahrungsmittelprodukt eine Basis aus geröstetem Brot enthält, der Anteil der Zubereitungsformulierung zwischen 2 % und 4 % bezogen auf das Gewicht der gesamten Produktzusammensetzung, einschließlich der Grenzen, liegt; und wenn das Nahrungsmittelprodukt eine Basis aus speziellen Brotteig enthält, der Anteil der Zubereitungsformulierung zwischen 2,5 % und 4 % bezogen auf das Gewicht der gesamten Produktzusammensetzung, einschließlich der Grenzen, liegt.

15. Verwendung der Nahrungsmittelzubereitungsformulierung nach einem der Ansprüche 1 bis 3 als ein Nahrungsmittelzusatz.

## Revendications

1. Une formulation alimentaire, **caractérisée en ce qu'**elle comprend des micro-algues fraîches sous forme de pâte de l'espèce *Chlorella vulgaris* dans une proportion comprise entre 14 % et 19 % du poids total de la formulation et avec un degré moyen d'humidité à température ambiante compris entre 81 % et 86 %, valeurs limite toutes deux incluses, et **en ce que** les micro-algues de l'espèce *Chlorella vulgaris* sont de la souche *Chlorella vulgaris fo. tertia* avec le numéro de dépôt CCAP 211/11D.

2. La formulation alimentaire selon la revendication 1, qui consiste en une pâte de micro-algues fraîches de l'espèce *Chlorella vulgaris,* dans une proportion comprise entre 14 % et 19 % du poids total de la formulation et avec un degré moyen d'humidité à température ambiante compris entre 81 % et 86 %, valeurs limite toutes deux incluses, et dans laquelle les micro-algues de l'espèce *Chlorella vulgaris* sont de la souche *Chlorella vulgaris fo. tertia* avec le numéro de dépôt CCAP 211/11D.

3. La formulation alimentaire selon l'une quelconque des revendications 1 ou 2, dans laquelle les micro-algues fraîches sont décolorées et désodorisées.

4. Un procédé d'obtention de la formulation alimentaire décrite dans l'une quelconque des revendications 1 à 3, **caractérisé en ce qu'**il comprend le fait de soumettre une culture aqueuse de micro-algues fraîches de l'espèce *Chlorella vulgaris* à une séparation mécanique solide-liquide pour former une pâte de micro-algues ayant une teneur en micro-algues comprise entre 14 % et 19 % du poids total de la formulation et un degré d'humidité compris entre 81 % et 86 %, valeurs limite toutes deux incluses, et dans lequel les micro-algues de l'espèce *Chlorella vulgaris* sont de la souche *Chlorella vulgaris fo. tertia* avec le numéro de dépôt CCAP 211/11D.

5. Le procédé selon la revendication précédente, dans laquelle la séparation mécanique solide-liquide est effectuée par centrifugation.

6. Le procédé selon l'une quelconque des revendications 4 ou 5, qui comprend le fait de cultiver et de récolter les micro-algues qui sont soumises à une séparation mécanique pour former la pâte.

7. Le procédé selon l'une des revendications 4 à 6, consistant à décolorer et/ou désodoriser les micro-algues.

8. Le procédé selon la revendication précédente, dans laquelle la désodorisation est effectuée par congélation de la culture avant de la soumettre à une séparation mécanique solide-liquide, à une température comprise entre -18°C et -20°C pendant une durée comprise entre 12 et 24 heures, valeurs limite toutes deux incluses.

9. Un produit alimentaire, **caractérisé en ce qu'**il comprend dans sa composition la formulation décrite dans l'une quelconque des revendications 1 à 3.

10. Le produit alimentaire selon la revendication précédente, qui est choisi dans le groupe constitué par les produits de panification, les produits de boulangerie et les produits de confiserie.

11. Le produit alimentaire selon l'une quelconque des revendications 9 ou 10, qui comprend la formulation en un pourcentage compris entre 0,05 % et 5 % en poids du total de la composition du produit.

12. Le produit alimentaire selon l'une des revendications 10 ou 11, qui est choisi dans le groupe constitué par les produits à base de pâte à frire, à base de pâte à pâtisserie, à base de pâte fermentée, à base de pâtes sèches, à base de feuilles de génoise et à base de pâte à pain spéciale et de produits de boulangerie frits.

13. Le produit alimentaire selon la revendication précédente, dans lequel les produits à base de pâte à frire sont sélectionnés dans le groupe constitué par les petits gâteaux, les génoises "sobao", les génoises sans levain, les muffins, les "gloria" et les biscuits mous sucrés ; les produits à base de pâte à pâtisserie sont sélectionnés dans le groupe constitué par les feuilletés, les tartes aux pommes, les "lazo", les "triangulo" et les millefeuilles ; les produits à base de pâte fermentée sont sélectionnés dans le groupe constitué par le pain grillé, les croissants, le "pandorino", le pain brioché et l'"ensaimada"; les produits à base de pâtes sèches sont sélectionnés dans le groupe constitué par les "tortas", les gâteaux aux amandes et les "rosegón" ; les produits à base d'une feuille de génoise sont sélectionnés dans le groupe des gâteaux de type "swiss-roll" ; les produits à base de pâte à pain spéciale sont choisis dans le groupe constitué par les pains tranchés, les pains à hamburger et les pains à hot-dogs ; et les pâtisseries frites sont choisies dans le groupe constitué par les beignets et les beignets fourrés.

14. Le produit alimentaire selon l'une quelconque des revendications 12 ou 13, dans lequel, lorsque ledit produit alimentaire contient une base de pâte à frire, la quantité de la formulation est comprise entre 2 % et 4 % en poids du total de la composition du produit, les deux valeurs limite étant incluses ; lorsque ledit produit alimentaire contient une base de pâte fermentée, la quantité de la formulation est comprise entre 2 % et 5 % en poids du total de la composition du produit, les deux valeurs limite étant incluses ; lorsque le produit alimentaire contient une base de pain grillé, la quantité de la formulation est comprise entre 2 % et 4 % en poids du total de la composition du produit, les deux valeurs limite étant incluses ; et lorsque le produit alimentaire contient une base de pâte à pain spéciale, la quantité de la formulation se situe entre 2,5 et 4 % en poids du total de la composition du produit, les deux valeurs limite étant incluses.

15. Utilisation de la formulation alimentaire décrite dans l'une des revendications 1 à 3 en tant qu'additif alimentaire.
